# EUROPEAN PATENT SPECIFICATION

(11) **EP 0 618 795 B1**
(45) Date of publication and mention of the grant of the patent: **09.06.1999**
(21) Application number: 93921926.7
(22) Date of filing: 07.10.1993
(51) Int. Cl.: A61K 7/48, A61K 9/00

(54) **PREPARATIONS CONTAINING A FLUOROCARBON EMULSION AND USABLE AS COSMETICS OR DERMATICS**
PRÄPARATE MIT FLUORKOHLENWASSERSTOFFEMULSIONEN VERWENDBAR ALS KOSMETIKA UND DERMATIKA
PREPARATIONS CONTENANT UNE EMULSION DE FLUOROCARBONE ET UTILISABLES DANS DES PRODUITS COSMETIQUES OU DERMIQUES

(30) Priority: 29.10.1992 DE 4236607
(43) Date of publication of application: 12.10.1994
(73) Proprietor: LANCASTER GROUP GmbH, 55116 Mainz (DE)
(72) Inventor: ZASTROW, Leonhard, D-65205 Wiesbaden-Nordenstadt (DE); STANZL, Klaus, D-56323 Waldesch (DE); RÖDING, Joachim, D-65207 Wiesbaden (DE)
(74) Representative: Walter, Wolf-Jürgen
(86) International application number: EP9302751
(87) International publication number: WO9409754

(56) References cited:
- WO-A-91/00110
- DE-A- 2 534 315

## Description

The invention relates to cosmetic and dermatological preparations which are efficient in improving the oxygen supply to the skin.

To improve supply of oxygen to the skin, it has already been proposed to use peroxides, such as hydrogen peroxide, in order to stimulate the cell metabolism of the skin via the nascent oxygen formed. However, the considerable side effects, such as skin irritations, have prevented their use. DE-A-2,534,315 claims an O₂-containing cosmetological formulation composed of an O₂-saturated gaseous fluorocarbon and a surfactant in aqueous phase in an aerosol container. Borgarello (EP-A-296,661) has developed an isotropic single-phase system for the cosmetic sector in which halogenated compounds are supposed to act as oxygen carriers. A typical composition consists of 34% of a mixture of perchloro-1-butyltetrahydrofuran and polyfluoro-1-propyltetrahydrofuran, 7% of isopropanol, 49% of water and 10% of emulsifier. The emulsifiers used are highly surface-active fluorine surfactants, for example of the perfluoroalkanesulfonamide type, which are known to be extremely toxic on i.p. application in mice (LD50 0.1 to 0.2 g/kg) and also act as an irritant on the skin. Further possible solutions relate to the use of a haemolymph extract from crustaceans or of an extract of proteins and proteids from bovine spleen. Human haemoglobin or, more recently, haemoglobin prepared by biotechnology has hitherto not been available in a form utilizable for topical purposes. Furthermore, it is known that PO/EO block polymers having EO contents of up to 50% have relatively good solubility for gaseous oxygen. It, contrast, block polymers having EO contents of 60 to 80% are solid products exhibiting low solubility for oxygen. WO-A-9100110 refers to fluorocarbon emulsions employing an ionic emulsifying agent with essentially saturated carbon bounds and their use as blood substitutes for intravenous use and as emollients for burns.

However, so far no convincing and physically detectable toning and reviving of the skin surface could be achieved by means of the preparations and methods mentioned.

The object of the invention is to improve the oxygen supply to the skin by means of a specific preparation based on a suitable emulsion of fluorocarbons in such a manner that a detectable effect is achieved.

According to the invention, the preparation, selected from the group consisting of a powder, a lotion, a shampoo, a cream or a gel paste, consists of an aqueous emulsion having a nonionic surfactant content of 1 to 8% by weight as the emulsifier and an oxygen-loaded fluorocarbon or fluorocarbon mixture content, the fluorocarbon content being in the range from 40 to 100% w/v (w/v = weight/volume) in a carrier suitable for the application and wherein the critical solubility temperature (in n-hexane) is a feature for selection of the fluorocarbons or fluorocarbon mixtures.

The preparation according to the invention can be present, depending on the specific method of application, in solid, liquid or semi-liquid form as powder, lotion, shampoo, cream or gel paste. The carriers suitable for each of these forms are known to one skilled in the art.

The essential feature of the invention is the presence of the fluorocarbon emulsion in the preparations, which ensures efficient toning of the skin.

A multiplicity of fluorocarbons can be used, for example aliphatic straight-chain and branched fluoroalkanes, mono- or bicyclic and unsubstituted or fluoroalkyl-substituted fluorocycloalkanes, perfluorinated aliphatic or bicyclic amines, bis(perfluoroalkyl)ethenes, perfluoropolyethers or mixtures thereof. Particularly preferred fluorocarbons are perfluorodecaline, F-butyltetrahydrofuran, perfluorotributylamine, perfluorooctyl bromide, bis(fluorobutyl)ethene or bis(fluorohexyl)ethene or C₆-C₉-perfluoroalkane.

The fluorocarbon content in the emulsion is in the range from 40 to 100% by weight/volume. A particularly preferred range is that from 70 to 100% by weight/volume.

The term "fluorocarbons" used here is understood to mean perfluorinated or highly fluorinated carbon compounds or mixtures capable of transporting gases, such as O₂ and CO₂. Highly fluorinated hydrocarbon compounds for the purposes of this invention are those in which most hydrogen atoms have been replaced by fluorine atoms. In most cases, this is achieved if about up to 90% of the hydrogen atoms have been replaced by fluorine atoms. Preferred fluorocarbons for the purposes of the present invention are those in which at least 95% of the hydrogen atom have been replaced, 98% being more preferred and 100% being most preferred.

Suitable emulsifiers are nonionic surfactants, such as emulsifiers of the EO/PO block polymer type, fluorinated nonionic surfactants according to DD-WP131,555 or perfluorinated iminobis(polyoxyalkylene) according to DD-WP265,398 Al.

These fluorocarbon emulsions can be prepared by DD-WP222,494 A1 or another known method ("Tenside Surfactants Detergents", 25 (1989), 5, p. 284). The auxiliary emulsifiers used include alkali metal salts of long-chain fatty acids and phospholipids.

The advantage of the preparations according to the invention containing a fluorocarbon emulsion when used as cosmetics or dermatics is that an additional oxygen supply imparted by the fluorocarbon promotes circulation and thus the metabolic processes in the epidermal layer and activates the general state of transpiration. The increased cell respiration strengthens the natural skin defence potential and promotes elimination of skin toxins.

In contrast to the known preparations mentioned at the beginning, the preparations according to the invention demonstrate that owing to their extraordinarily high dissolving power for oxygen, the chemically inert fluorocarbons are capable of supplying the skin advantageously with oxygen in topical application.

The use as a cosmetic is not limited to parts of the human face but applies to all epidermal regions of the body, including cellulitis-affected fat tissue with a poor supply and the region of the scalp. Fluorocarbons themselves are chemically and biologically inert organic liquids having a high dissolving power for oxygen. Owing to these characteristics, they have been proposed as gas carriers in blood substitute emulsions and have also been used in humans (K.C. Lowe: "Blood substitutes", Ellis-Horwood, Chichester, GB, 1988).

Depending on the intended specific purpose, the fluorocarbons can be selected in accordance with O₂ solubility, partial vapour pressure and lipid solubility. The critical solubility temperature (CST) of the fluorocarbons in n-hexane correlates with their solubility in lipids, for example cell membranes, and is thus a measure of the rate of release through the skin. Thus, for example, perfluorodecaline and perfluorooctyl bromide which have small CST values are released fairly rapidly, while, on the other hand, F-tributylamine which has a high CST value of 59°C also exhibit a long half life of release. It has been found that fluorocarbons behave ideally upon mixing, and the CST values of such mixtures show a linear relationship with composition. By mixing various fluorocarbons, it is thus possible to establish defined CST values which often cannot be achieved by individual compounds. This finding makes it possible to use fluorocarbon mixtures selectively in order to positively affect the rate of penetration of the skin and their residence time.

Incorporation of the fluorocarbon emulsion as active component in ointments, creams, lotions and other aqueous or alcoholic cosmetic formulations is carried out as a function of the intended purpose, it being possible for the fluorocarbon content and thus the O₂ availability to be varied within wide limits. Before incorporation in any cosmetic systems, such as, for example, gels, pastes, powders, ointments, creams, lotions and waters or alcoholic extracts, the emulsion can be partially loaded or saturated with gaseous oxygen. Saturation with atmospheric oxygen already ensures a higher oxygen capacity than any comparable known systems by virtue of the equilibrium which is usually established in accordance with Henry's law.

According to the invention, the fluorocarbon emulsion content in the cosmetic or dermatological preparations can be in the range from 0.05 to 80% by weight, preferably in the range from 0.05 to 60% by weight, and in particular in the range from 1 to 50% by weight.

## Claims

1. Carrier-based cosmetic preparation containing a fluorocarbon emulsion characterized in that the preparation selected from the group consisting of a powder, a lotion, a shampoo, a cream or a gel paste, contains an aqueous emulsion comprising (i) 1 - 8 % by weight of an nonionic surfactant as the emulsifier selected from the group consisting of perfluorinated iminobis(polyoxyalkylenes), polyoxyethylene/polyoxypropylene copolymers, ethoxylated fluorine surfactants and ethoxylated polypropylene glycols, and (ii) an oxygen-loaded fluorocarbon or fluorocarbon mixture, the fluorocarbon content being in the range from 40 to 100 % w/v and wherein the critical solubility temperature (in n-hexane) is a feature for selection of the fluorocarbons or fluorocarbon mixtures.

2. Preparation according to claim 1, characterized in that the fluorcarbon content is in the range from 70 to 100 % w/v.

3. Preparation according to claim 1, characterized in that it contains the oxygen loaded emulsion of the fluorocarbone(s) in an amount of 0.05 to 80 % by weight, preferably in an amount of 0.05 to 60 % by weight, and in particular in an amount of 1 to 50 % by weight.

4. Preparation according to one of the claims 1 to 3, characterized in that the fluorocarbons are selected from the group selected from aliphatic straight-chain and branched fluoroalkanes, mono- or bicyclic, unsubstituted or fluoroalkyl-substituted fluorocycloalkanes, perfluorinated aliphatic or bicyclic amines, bis(perfluoroalkyl)ethenes and mixtures thereof.

5. Preparation according to claim 4, characterized in that the fluorocarbons are selected from the group consisting of perfluorodecalin, F-butyltetrahydrofuran, perfluorotributylamine, perfluorooctyl bromide, bis-fluoro(butyl)ethene or C₆-C₉-perfluoroalkanes.

6. Use of a preparation according to one of the claims 1 to 5 as cosmetic for controlling the oxygen supply to the skin.

## Patentansprüche

1. Kosmetische Zubereitung auf Basis von Trägerstoffen und eine Fluorcarbonemulsion enthaltend, dadurch gekennzeichnet, daß die Zubereitung, ausgewählt aus der Gruppe, bestehend aus einem Puder, einem Shampoo, einer Creme, oder einer Gelpaste, eine wäßrige Emulsion enthält, umfassend
(i) 1-8 Gew% eines nichtionogenen Tensids als Emulgator, ausgewählt aus der Gruppe, bestehend aus perfluorierten Imino-Bis(polyoxyalkylenen), Polyoxyethylen-Polyoxypropylen-Copolymeren, ethoxylierten Sorbitanfettsäureestern und ethoxylierten Polypropylenglykolen, und
(ii) ein mit Sauerstoff beladenes Fluorcarbon oder Fluorcarbongemisch, wobei der Anteil an Fluorcarbon im Bereich von 40 bis 100% w/v (Gewicht/Volumen) liegt und worin die kritische Löslichkeitstemperatur (in n-Hexan) ein Merkmal zur Auswahl der Fluorcarbone oder Fluorcarbongemische ist.

2. Zubereitung nach Anspruch 1, dadurch gekennzeichnet, daß der Fluorcarbongehalt im Bereich von 70 bis 100 w/v liegt.

3. Zubereitung nach Anspruch 1, dadurch gekennzeichnet, daß sie die mit Sauerstoff beladene Emulsion des (der) Fluorcarbon(e) in einer Menge von 0,05 bis 80 Gew%, vorzugsweise in einer Menge von 0,05 bis 60 Gew% und insbesondere in einer Menge von 1 bis 50 Gew% enthält.

4. Zubereitung nach Anspruch 1 bis 3, dadurch gekennzeichnet, daß die Fluorcarbone aus der Gruppe ausgewählt sind, die aus aliphatischen geradkettigen und verzweigten Fluoralkanen, mono- oder bicyclischen, gegebenenfalls fluoralkylsubstituierten Fluorcycloalkanen, perfluorierten aliphatischen oder bicyclischen Aminen, Bis-(perfluoralkyl)-ethenen oder deren Gemischen ausgewählt sind.

5. Zubereitung nach Anspruch 4, dadurch gekennzeichnet, daß die Fluorcarbone aus der Gruppe ausgewählt sind, die aus Perfluordecalin, F-Butyltetrahydrofuran, Perfluortributylamin, Perfluoroctylbromid, Bis-Fluor(butyl)ethen oder C₆-C₉-Perfluoralkanen besteht.

6. Verwendung einer Zubereitung nach einem der Ansprüche 1 bis 5 als Kosmetikum zur Steuerung der Sauerstoffversorgung der Haut.

## Revendications

1. Préparation cosmétique a base de substances supports et contenant une émulsion de fluorocarbone, caractérisée en ce que la préparation, sélectionnée dans le groupe comprenant une poudre, un shampooing, une crème ou un gel, renferme une émulsion aqueuse comprenant:
(i) 1-8 % en poids d'un agent de surface non ionogène en tant qu'émulsifiant, sélectionné dans le groupe comprenant les imino-bis(polyoxyalkylènes), les copolymères polyoxyéthylène-polyoxypropylène, les esters d'acide gras de sorbitanne éthoxylés et les polypropylèneglycols, et
(ii) un fluorocarbone ou mélange de fluorocarbone chargé en oxygène, la proportion de fluorocarbone se situant dans l'intervalle de 40 à 100 % p/v (poids/volume), et dans lequel la température de solubilité critique (dans du n-hexane) est une caractéristique présidant au choix des fluorocarbones ou mélanges de fluorocarbones.

2. Préparation selon la revendication 1, caractérisée en ce que la teneur en fluorocarbone se situe dans l'intervalle de 70 à 100 p/v.

3. Préparation selon la revendication 1, caractérisée en ce qu'elle renferme l'émulsion du (des) fluorocarbone(s) chargé(s) en oxygène, en une quantité de 0,05 à 80 % en poids, de préférence en une quantité de 0,05 à 60 % en poids, et en particulier en une quantité de 1 à 50 % en poids.

4. Préparation selon les revendications 1 à 3, caractérisée en ce que les fluorocarbones sont sélectionnés dans le groupe comprenant les fluoroalcanes aliphatiques à chaîne linéaire et ramifiés, les fluorocycloalcanes mono- ou bicycliques, éventuellement substitués fluoroalkyle, les amines aliphatiques ou bicycliques perfluorées, les bis-(perfluoroalkyl)-éthènes ou leurs mélanges.

5. Préparation selon la revendication 4, caractérisée en ce que les fluorocarbones sont sélectionnés dans le groupe comprenant la perfluorodécaline, le F-butyltétrahydrofurane, la perfluorotributylamine, le perfluoro-octylbromure, le bis-fluor(butyl)éthène ou les perfluoroalcanes en C₆-C₉.

6. Utilisation d'une préparation selon l'une des revendications 1 à 5, comme cosmétique pour le contrôle de l'oxygénation de la peau.
